Europäisches Patentamt

European Patent Office

Office européen des brevets

(11) Veröffentlichungsnummer: **0 287 961 B1**

## (12) EUROPÄISCHE PATENTSCHRIFT

(45) Veröffentlichungstag der Patentschrift: **12.07.95**

(21) Anmeldenummer: **88106008.1**

(22) Anmeldetag: **15.04.88**

(51) Int. Cl.⁶: **C07H 1/08**, C07H 21/00, C12P 19/34

## (54) Verfahren zur Isolierung von Nukleinsäuren.

(30) Priorität: **24.04.87 CH 1573/87**

(43) Veröffentlichungstag der Anmeldung:
**26.10.88 Patentblatt 88/43**

(45) Bekanntmachung des Hinweises auf die
Patenterteilung:
**12.07.95 Patentblatt 95/28**

(84) Benannte Vertragsstaaten:
**AT BE CH DE FR GB IT LI NL SE**

(56) Entgegenhaltungen:
**CH-A- 263 036**
**DE-A- 1 617 498**

**T. MANIATIS et al.: "Molecular Cloning - A Laboratory Manual" COLD SPRINGHARBOR LABORATORY,1982 Seiten 458-463**

(73) Patentinhaber: **F. HOFFMANN-LA ROCHE AG**
**Postfach 3255**
**CH-4002 Basel (CH)**

(72) Erfinder: **Kiefer, Hansruedi, Dr.**
**Habermarkweg 7**
**CH-4125 Riehen (CH)**

(74) Vertreter: **Wächter, Dieter Ernst, Dr. et al**
**F.Hoffmann-La Roche AG**
**Grenzacherstrasse 124**
**Postfach 3255**
**CH-4002 Basel (CH)**

**Beschreibung**

Bei der Isolierung von Nukleinsäuren, wie z.B. Desoxyribonukleinsäuren (DNA) oder Ribonukleinsäuren (RNA), aus biologischem Gewebe oder aus Zellen, z.B. für Forschungszwecke oder für diagnostische Zwecke, müssen diese von den übrigen Gewebe- bzw. Zellbestandteilen, wie z.B. Proteinen, Lipiden, etc., abgetrennt werden. Zu diesem Zweck kann zuerst ein Gewebe- oder Zellextrakt hergestellt werden, aus dem dann die Nukleinsäuren in einem Zwei-Phasensystem abgetrennt werden können (Mainwaring et al., in "Nucleic Acid Biochemistry and Molecular Biology", Blackwell Scientific Publications Oxford (GB), p. 75-76 [1982]). Die folgenden zwei Methoden sind die gebräuchlichsten, wobei sie auch kombiniert werden können (Maniatis et al., in "Molecular Cloning - A Laboratory Manual", Cold Spring Harbor Laboratory, p. 458-460 [1982]). Bei der einen Methode verwendet man ein Wasser/Phenol-Zwei-Phasensystem, bei dem Proteine und Peptide in der organischen Phase ausgefällt bzw. gelöst werden, während Nukleinsäuren in der wässrigen Phase verbleiben, sofern eine bestimmte Salzkonzentration und neutrales pH eingehalten werden. Bei der zweiten Methode handelt es sich um die Chloroform/Isoamylalkohol bzw. Chloroform/Oktanol-Methode, bei der die Nukleinsäuren ebenfalls in der wässrigen Phase verbleiben, während die denaturierten andern Makromoleküle an der Grenzschicht zwischen den Phasen ausgeschieden werden.

Wesentlich für die Isolierung von Nukleinsäuren nach diesen Methoden mit Zwei-Phasensystemen ist, dass wässrige und organische Phase zuerst gründlich durchmischt und anschliessend wieder getrennt werden müsen. Um eine schnellere Phasentrennung zu erreichen, wird üblicherweise zentrifugiert. Bei der Isolation von intakter chromosomaler Desoxyribonukleinsäure (DNA) muss zusätzlich darauf geachtet werden, dass beim Mischen die DNA nicht durch Scherkräfte in kleine Bruchstücke gespalten wird.

Nach der Phasentrennung wird die organische Phase abgetrennt und, falls nötig, die wässrige Phase erneut mit einem gleichen Volumen Phenol, Chloroform/Isoamylalkohol oder Chloroform/Oktanol extrahiert. Die Extraktion der wässrigen Phase mit der organischen Phase wird solange durchgeführt, bis die Nukleinsäuren in reiner Form mit Alkohol (z.B. Aethanol oder Isopropanol) aus der wässrigen Phase ausgefällt werden können. Allerdings benötigt die Extraktion von Nukleinsäuren auf die obenbeschriebene Art und Weise, bedingt durch die mehrfachen Misch- und Zentrifugationszyklen, einen grossen Zeitaufwand.

Es wurde nun gefunden, dass Nukleinsäuren überraschenderweise auch durch einfache Fällung mit einem wasserlöslichen Keton, vorzugsweise mit Aceton aus einem, vorzugsweise mit Proteasen behandelten, Zell- oder Gewebeextrakt isoliert werden können. Die ausgefällten Nukleinsäuren können durch Filtration oder Zentrifugation abgetrennt werden. Die so isolierten Nukleinsäuren können, falls nötig, mehrmals mit Alkohol, vorzugsweise mit 70% v/v Aethanol in Wasser gewaschen werden. Gegebenenfalls kann das Nukleinsäure-Präzipitat auch vor der Waschung mit Alkohol nochmals in Wasser, oder einer Pufferlösung, z.B. in STET-Puffer enthaltend 8% Saccharose, 5% Triton™ X-100, 50 mM Aethylendiamintetraessigsäure-Dinatriumsalz (EDTA) und 50 mM Tris/HCl (pH 8.0), gelöst und anschliessend erneut mit Aceton gefällt werden.

In der Schweizerischen Patentschrift CH-A-263 036 wird zwar die Verwendung von Aceton bei der Extraktion von pflanzlichem, bzw. tierischem Material, das an Proteine gebundene Nukleotide enthält, beschrieben. Das Aceton wird in dem Verfahren gemäss der CH-A-263 036 aber zur Fällung der Proteine und nicht der Nukleinsäuren verwendet.

Die Deutsche Offenlegungsschrift DE-A-1 617 498 offenbart ein Verfahren zur Herstellung einer Nukleinsäuresubstanz. Da diese Nukleinsäuresubstanz aber unbeeinflusst ist von Desoxyribonuklease, unterscheidet sich diese Substanz klar von der hochmolekularen DNA, die gemäss dem Verfahren der vorliegenden Erfindung erhalten wird, da diese hochmolekulare DNA durch DNase schnell aufgebaut wird. Die Acetonbehandlung gemäss der DE-A-1 617 498 dient einzig der Bereitstellung eines Acetonpulvers einer Pilzsuspension und nicht der direkten Isolierung der DNA aus einem wässerigen Zell- oder Gewebeextrakt.

Die vorliegende Erfindung betrifft daher ein Verfahren zur Isolierung von Nukleinsäuren, vorzugsweise von Desoxyribonukleinsäure, aus einem Zell- oder Gewebeextrakt, dadurch gekennzeichnet, dass

a) der Zell- oder Gewebeextrakt in Gegenwart von Detergenzien mit einem Überschuss einer wässerigen Lösung, die mindestens 60 Volumenprozente Aceton oder ein ähnliches, wasserlösliches Keton wie z.B. 2-Butanon, 2-Pentanon, 3-Pentanon oder 3-Methylcyclopentanon, enthält, vermischt wird; und

b) die nach der Durchmischung ausgefällte Nukleinsäure aus der Mischung abgetrennt wird.

Das erfindungsgemässe Verfahren eignet sich zur Isolation von Nukleinsäuren, vorzugsweise von hochmolekularer DNA aus einem Zell- oder Gewebeextrakt von Pro- oder Eukaryonten. Die hochmolekulare DNA kann chromosomale oder extrachromosomale DNA sein. Beispiele für extrachromosomale DNA sind Plasmid-DNA, Virus-DNA oder mitochondriale DNA.

Zell- und Gewebeextrakte können hergestellt werden, indem die Zell- oder Gewebestruktur entweder mechanisch, chemisch oder enzymatisch aufgebrochen wird. Dabei können die klassischen Methoden der Biochemie verwendet werden (vergleiche z.B. Mahler et al., Biological Chemistry, New York, pp. 389-403 [1969] Harper & Row, New York). Gegebenenfalls können auch subzelluläre Fraktionen wie zum Beispiel eine Zytoplasma- oder eine Zellkern-Fraktion als Ausgansmaterial verwendet werden. Zur Vermeidung von DNA-Abbau durch Nukleasen können diese mit Detergentien (z.B. Triton™ X-100, Natriumdodecylsulfat (SDS), etc.), vorzugsweise mit SDS, denaturiert und die Proteine, inklusive der Nukleasen, durch Proteinasen (z.B. Pronase, Proteinase K, oder eine andere unspezifische Proteinase die in Gegenwart von Detergentien aktiv ist) abgebaut werden (Reaktionsbedingungen siehe Maniatis et al., supra).

Die Proteinase-Behandlung kann bei Raumtemperatur oder bei einer anderen Temperatur durchgeführt werden. Vorzugsweise wird bei einer Temperatur gearbeitet, die für die Aktivität der Proteinase optimal ist, d.h. bei Verwendung der Proteinase K oder der Pronase bei einer Temperatur von etwa 37°C bis 45°C. Proteinase K ist auch bei einer Temperatur von 65°C noch aktiv (Maniatis et al, supra, p. 85). Der so erhaltene Zell- oder Gewebeextrakt kann gegebenenfalls verdünnt werden und die Nukleinsäuren durch Zugabe von wasserlöslichem Keton, vorzugsweise Aceton, ausgefällt werden. Um die Fällung der Nukleinsäuren zu gewährleisten, muss eine Mindestmenge wasserlösliches Keton verwendet werden. Andererseits darf die Menge wasserlösliches Keton nicht so gross sein, bzw. der Wasseranteil nicht so klein sein, dass weitere Bestandteile des Zell- oder Gewebeextraktes, wie z.B. Peptide, Proteine, etc., ausgefällt werden. Die optimale Menge wasserlösliches Keton, die zur Fällung der Nukleinsäuren, aber nicht der andern Bestandteile des Zell- oder Gewebeextraktes, benötigt wird, kann durch den Fachmann leicht ermittelt werden. Vorzugsweise werden die Nukleinsäuren, insbesondere Desoxyribonukleinsäuren, durch Zugabe eines Ueberschusses an mindestens 60%igem wässrigen Aceton, bezogen auf das Volumen Zell- oder Gewebeextrakt, ausgefällt. Besonders bevorzugt ist die Zugabe von 10 Volumenteilen 70%igem, wässrigem Aceton. Zum Beispiel kann ein Teil Zell- oder Gewebextrakt, enthaltend etwa 0.6 bis 300 $\mu$g DNA in 0.5 bis 3 ml STET-Puffer, mit 10 Teilen einer mindestens 60%igen, bevorzugt einer 70%igen Aceton/Wasser-Lösung (v/v) versetzt werden. Anstelle von Aceton kann auch ein ähnliches, wasserlösliches Keton wie zum Beispiel 2-Butanon, 2-Pentanon, 3-Pentanon oder 3-Methylcyclopentanon verwendet werden. Bevorzugtes Keton ist jedoch Dimethylketon oder Aceton.

Zur Erhöhung der Löslichkeit von Zellbestandteilen, die nicht Nukleinsäuren sind, kann die wässrige Acetonlösung gegebenenfalls noch weitere organische Lösungsmittel enthalten, vorausgesetzt, dass die Ausfällung der Nukleinsäuren durch eine solche Zugabe nicht behindert wird. Zum Beispiel bewirkt die Zugabe von bis zu 0,1 Volumenteilen, vorzugsweise von 0,05 Volumenteilen Dimethylformamid oder Formamid zur wässrigen Acetonlösung eine Erhöhung der Löslichkeit von kleinen Peptiden. Schlecht wasserlösliche Peptide werden dadurch in Lösung gehalten und die Nukleinsäuren werden in reinerer Form ausgefällt. Andererseits bewirkt die Zugabe von Dimethylformamid oder Formamid eine Verlangsamung der Präzipitation, Dies hat zur Folge, dass die Nukleinsäuren in noch reiner Form präzipitieren. Bei der Fällung von Nukleinsäuren aus verdünnten Lösungen kann gegebenenfalls auf die Zugabe von Dimethylformamid oder Formamid verzichtet werden, um die Fällung der Nukleinsäuren nicht zu stark zu verlangsamen.

Die Ausfällung der Nukleinsäuren erfolgt spontan nach Zugabe der Acetonlösung. Gegebenenfalls kann das Gefäss, das die Mischung enthält, leicht bewegt werden, z.B. durch eine schaukelnde Bewegung.

Das Gefäss, das beim erfindungsgemässen Verfahren verwendet wird, sollte zumindest für die Dauer des Verfahrens gegen die verwendeten Lösungsmittel resistent sein, vorzugsweise werden Gefässe aus Glas, Teflon® oder Polypropylen verwendet.

Die ausgefällte Nukleinsäure kann abfiltriert oder auf andere übliche Art und Weise, zum Beispiel durch Zentrifugation und anschliessende Entfernung des Ueberstandes, von der Lösung abgetrennt werden.

Je nach Anforderungen bezüglich der Reinheit können die Nukleinsäuren direkt weiter verwendet werden oder einer weiteren Reinigung unterzogen werden. Zur weiteren Reinigung können die Nukleinsäuren erneut in einer Pufferlösung gelöst werden und wie oben beschrieben oder mit Alkohol z.B. mit Aethanol oder Isopropanol wie von Maniatis et al. (supra, pp. 461-462) beschrieben, nochmals gefällt werden. Die Fällung kann ein- oder mehrmals wiederholt werden. Die ausgefällten Nukleinsäuren können anschliessend mit Alkohol, vorzugsweise mit 70%-igem Aethanol (v/v) in Wasser und gegebenenfalls nochmals mit absolutem Alkohol, z.B. Aethanol gewaschen werden und gegebenenfalls im Vakuum oder unter einem leichten Stickstoffstrom getrocknet werden. Gegebenenfalls können die Nukleinsäuren zur weiteren Reinigung auch dialysiert werden (z.B. gegen 10 mM Tris/HCl (pH 7.8), 100 mM NaCl, 1 mM EDTA).

Das erfindungsgemässe Verfahren wird vorzugsweise bei Raumtemperatur oder in einer Kühlkammer (z.B. bei 4°C) durchgeführt.

DNA die mit dem erfindungsgemässen Verfahren isoliert wird, bleibt auch bei längerer Inkubation bei Raumtemperatur oder bei 37°C stabil. Sie kann durch Restriktionsenzyme spezifisch gespalten werden und ist biologisch aktiv.

Zur Bestimmung der Reinheit der erhaltenen Nukleinsäure kann das Verhältnis der UV-Absorption bei den Wellenlängen 260 nm und 280 nm gemessen werden.

Die folgenden Figuren und die nachfolgenden Beispiele illustrieren verschiedene Ausführungsformen der vorliegenden Erfindung, sollen sie aber in keiner Art und Weise beschränken.

## Fig. 1

Die DNA von je N x $10^6$ Zellen (Maus Monozyten-Makrophagenzellinie J774A.1, ATCC No. TIB 67), die zuvor während 2 Stunden mit [$^3$H]-Thymidin (5 Ci/mmol, 1 $\mu$Ci/ml Kulturmedium, 6 x $10^5$ Zellen/ml) markiert worden waren, wurde entweder direkt mit eiskalter Trichloressigsäurelösung auf Whatman® GF/C Filter präzipitiert oder gemäss Beispiel 1 isoliert. Die relative Ausbeute wurde durch Messung der Radioaktivität (cpm), der auf den Filter präzipitierten (X) bzw. gemäss Methode 1 isolierten DNA (●), bestimmt. Es zeigte sich, dass die DNA aus einer Zellzahl im Bereich von 1 x $10^6$ bis 1,6 x $10^7$ mit der erfindungsgemässen Methode praktisch quantitative isoliert werden kann.

## Fig. 2

Die DNA von je N x $10^6$ Zellen (Maus Monozyten-Makrophagenzellinie J774A.1, ATCC No. TIB 67) wurden gemäss Beispiel 1 isoliert und die erhaltene Menge DNA (optische Dichte bei 260 nm = $OD_{260}$) in Abhängigkeit von der Zellzahl (N x $10^6$), die bei der Extraktion eingesetzt wurde, dargestellt.

## Beispiel 1

1 x $10^7$ Zellen einer B-Zellinie wurden in einem Polypropylenröhrchen (Falcon 2059), 17 x 100 mm) durch Zugabe von 1 ml Lysepuffer (50 mM Tris/HCl (pH 9-10), 100 mM EDTA, 1% SDS) lysiert. Die Proteine wurden durch Zugabe von 100 $\mu$l einer 20 mg/ml Pronaselösung während 90 Minuten bei 37°C verdaut. Anschliessend wurde bei Raumtemperatur 10 ml Lösung P bestehend aus 95 Volumenprozenten 70%-iges Aceton in Wasser (v/v) und 5 Volumenprozenten Dimethylformamid (DMF) zugegeben. Das Gemisch wurde durch schaukelnde Bewegung des Röhrchens während 5 Minuten bei Raumtemperatur gut durchmischt. Dabei bilden die zunächst langen DNA-Fäden einen kleinen Knäuel. Die DNA wurde abfiltriert und zuerst 3mal mit 70%-igem Aethanol in Wasser, dann 2mal mit absolutem Aethanol gewaschen. Die DNA wurde mit einem Stickstoffstrom getrocknet und anschliessend in 1.0 ml Wasser gelöst. Ausbeute: 100 $\mu$g, $A_{260}/A_{280}$ = 1,8. Die isolierte DNA war über mehrere Tage stabil (Agarose-Gelelektrophorese gemäss Maniatis et al., supra, p. 150-161).

## Beispiel 2

100 ml einer Uebernacht-Kultur von E.coli K 802 (ATCC No. 33526) enthaltend das Plasmid pAG60 (Traunecker, Immunol. Methods, 3, 55-67 [1985]) wurden bei 4000 g während 10 Minuten bei 4°C zentrifugiert und die Zellen im Sediment gemäss der Methode von Maniatis et al. (supra, p. 89-91) lysiert. Das Zellysat (2 ml) wurde mit 10 ml Lösung P versetzt und die DNA wie in Beispiel 1 isoliert. Ausbeute: 200 $\mu$g, $A_{260}/A_{280}$ = 1.83. Die isolierte DNA war über mehrere Tage stabil und liess sich mit verschiedenen Restriktionsenzymen spalten.

## Beispiel 3

Die DNA von 1,5 x $10^7$ Zellen einer Makrophagen-Tumorzellinie wurde wie in Beispiel 1 beschrieben, unter Verwendung von 3 verschiedenen Zusammensetzungen der Lösung P, gefällt.

Versuch A: Lösung P =
70%iges Aceton in Wasser (v/v)
Versuch B: Lösung P =
95 Volumenprozente 70%iges Aceton in Wasser (v/v) und 5 Volumenprozente Dimethylformamid
Versuch C: Lösung P =
95 Volumenprozente 70%iges Aceton in Wasser (v/v) und 5 Volumenprozente Formamid.

Anschliessend wurde die DNA abfiltriert und dreimal entweder mit 70%igem Aethanol oder mit 70%igem Isopropanol gewaschen. Die getrocknete DNA wurde in 1 ml TE-Puffer (20 mM Tris/HCl (pH 7,6), 2 mM EDTA) gelöst. Es zeigte sich, dass in allen Versuchen die DNA praktisch quantitativ isoliert werden konnte und mit Restriktionsenzymen (z.B. mit EcoR1) verdaubar war. Die DNA war während mindestens 18 Stunden bei Inkubation bei 4°C, bei Raumtemperatur und bei 37°C stabil, was darauf hindeutet, dass die isolierte DNA frei von Nukleasen ist.

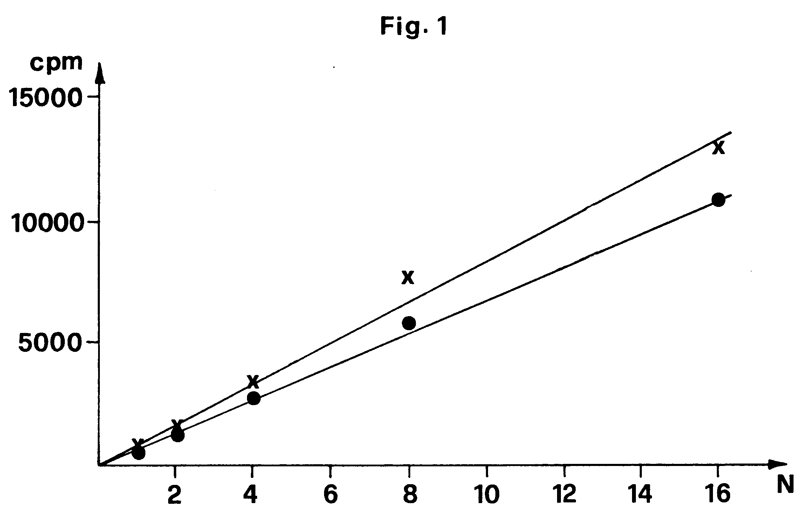

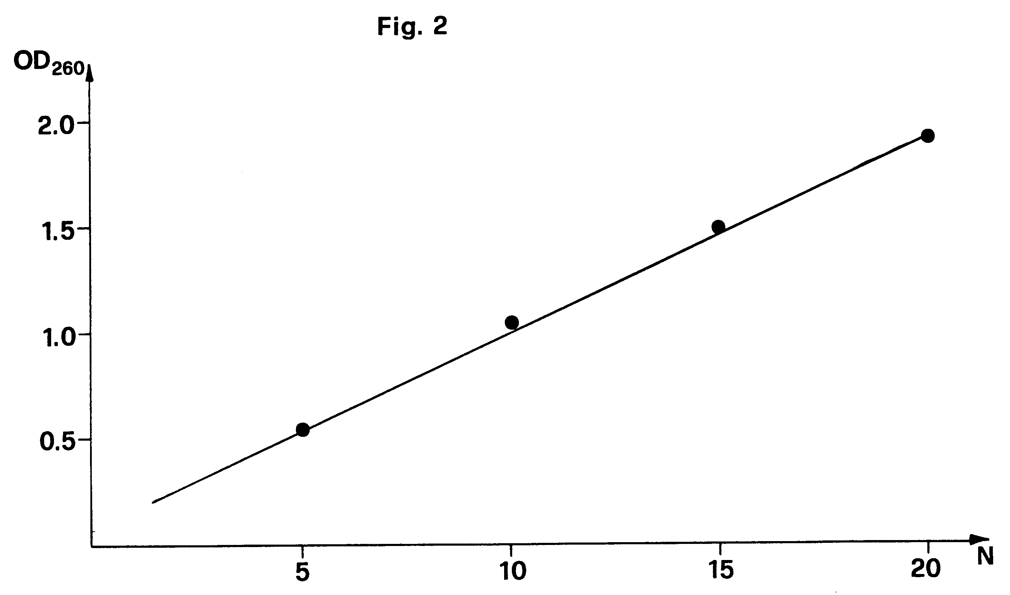

## Patentansprüche

1. Verfahren zur Isolierung von Nukleinsäuren aus einem Zell- oder Gewebeextrakt, dadurch gekennzeichnet, dass

a) der Zell- oder Gewebeextrakt in Gegenwart von Detergenzien mit einem Ueberschuss einer wässerigen Lösung, die mindestens 60 Volumenprozente Aceton oder ein ähnliches, wasserlösliches Keton wie z.B. 2-Butanon, 2-Pentanon, 3-Pentanon oder 3-Methylcyclopentanon, enthält, vermischt wird; und

b) die nach der Durchmischung ausgefällte Nukleinsäure aus der Mischung abgetrennt wird.

2. Verfahren nach Anspruch 1, dadurch gekennzeichnet, dass die isolierte Nukleinsäure Desoxribonukleinsäure ist.

3. Verfahren nach Anspruch 1 oder 2, dadurch gekennzeichnet, dass der Zell- oder Gewebeextrakt vorgängig mit einer nichtspezifischen Protease in Gegenwart von Detergenzien behandelt wird.

4. Verfahren nach Anspruch 1, 2 oder 3, dadurch gekennzeichnet, dass im Verfahrensschritt a) eine wässrige Acetonlösung, die mindestens 60 Volumenprozente Aceton enthält, verwendet wird.

5. Verfahren nach Anspruch 1, 2, 3 oder 4 dadurch gekennzeichnet, dass im Verfahrensschritt a) eine wässrige Acetonlösung, die mindestens 70 Volumenprozente Aceton enthält, verwendet wird, vorzugsweise in einem Ueberschuss von etwa 10 Volumenteilen Acetonlösung zu 1 Teil Zell- oder Gewebeextrakt.

6. Verfahren nach Anspruch 1, 2, 3, 4 oder 5 dadurch gekennzeichnet, dass die wässerige Lösung im Verfahrensschritt a) zusätzlich etwa 0,05 bis 0,1 Volumenteile Dimethylformamid, Formamid oder ein ähnliches aprotisches, polares Lösungsmittel enthält.

## Claims

1. A process for the isolation of nucleic acids from a cell or tissue extract, characterized by
a) mixing the cell or tissue extract in the presence of detergents with an excess of an aqueous solution which contains at least 60 percent by volume of acetone or an analogous, water-soluble ketone such as e.g. 2-butanone, 2-pentanone, 3-pentanone or 3-methylcyclopentanone; and
b) separating from the mixure the nucleic acid which precipitates after the intermixing.

2. A process according to claim 1, characterized in that the isolated nucleic acid is desoxyribonucleic acid.

3. A process according to claim 1 or 2, characterized in that the cell or tissue extract is previously treated with a non-specific protease in the presence of detergents.

4. A process according to claim 1, 2 or 3, characterized in that an aqueous acetone solution which contains at least 60 percent by volume of acetone is used in process step a).

5. A process according to claim 1, 2, 3 or 4, characterized in that an aqueous acetone solution which contains at least 70 percent by volume of acetone is used in process step a), preferably in an excess of about 10 parts by volume of acetone solution to 1 part of cell or tissue extract.

6. A process according to claim 1, 2, 3, 4 or 5, characterized in that the aqueous solution in process step a) additionally contains about 0.05 to 0.1 parts by volume of dimethylformamide, formamide or an analogous aprotic, polar solvent.

## Revendications

1. Procédé pour isoler des acides nucléiques d'un extrait cellulaire ou tissulaire, caractérisé en ce que
a) on mélange à l'extrait cellulaire ou tissulaire, en présence de détergents, un excès d'une solution aqueuse qui contient au moins 60 % en volume d'acétone ou d'une cétone analogue hydrosoluble telle par exemple que la 2-butanone, la 2-pentanone, la 3-pentanone ou la 3-méthylcyclopentanone ; et
b) on isole du mélange l'acide nucléique qui précipite après l'opération de mélange.

2. Procédé selon la revendication 1, caractérisé en ce que l'acide nucléique isolé est l'acide désoxyribonucléique.

3. Procédé selon la revendication 1 ou 2, caractérisé en ce que l'extrait cellulaire ou tissulaire est au préalable traité par une protéase non spécifique, en présence de détergents.

4. Procédé selon la revendication 1, 2 ou 3, caractérisé en ce qu'on utilise dans l'étape a) une solution aqueuse d'acétone contenant au moins 60 % en volume d'acétone.

5. Procédé selon la revendication 1, 2, 3 ou 4, caractérisé en ce qu'on utilise dans l'étape a) une solution aqueuse d'acétone contenant au moins 70 % en volume d'acétone, de préférence selon un excès d'environ 10 parties en volume de solution d'acétone pour 1 partie d'extrait cellulaire ou tissulaire.

6. Procédé selon la revendication 1, 2, 3, 4 ou 5, caractérisé en ce que la solution aqueuse, dans l'étape a), contient en outre d'environ 0,05 à 0,1 partie en volume de diméthylformamide, de formamide ou d'un solvant aprotique polaire analogue.

Fig. 1

Fig. 2

EP 0 287 961 B1